# EUROPÄISCHE PATENTSCHRIFT

(11) **EP 3 237 019 B1**
(45) Veröffentlichungstag und Bekanntmachung des Hinweises auf die Patenterteilung: **09.10.2019**
(21) Anmeldenummer: 16718844.0
(22) Anmeldetag: 07.04.2016
(51) Int. Cl.: A61K 49/00, A61K 47/32, A61K 9/00, A61K 9/08, A61K 31/121

(54) **FORMULIERUNG VON HYPERICIN ZUR PHOTODYNAMISCHEN DIAGNOSE**
FORMULATION OF HYPERICIN FOR PHOTODYNAMIC DIAGNOSIS
FORMULATION D'HYPÉRICINE POUR DIAGNOSTIC PHOTODYNAMIQUE

(30) Priorität: 28.09.2015 AT 6292015
(43) Veröffentlichungstag der Anmeldung: 01.11.2017
(73) Patentinhaber: Sanochemia Pharmazeutika AG, 1090 Wien (AT)
(72) Erfinder: ABRAHAMSBERG, Christina, 1030 Wien (AT); FRANTSITS, Werner, 1130 Wien (AT); GERDES, Klaus, 41564 Kaarst Vorst (DE); GUNGL, József, 9423 Ágfalva (HU); KÄLZ, Beate, 7035 Steinbrunn (AT); WELZIG, Stefan, 1030 Wien (AT)
(74) Vertreter: Beer & Partner Patentanwälte KG
(86) Internationale Anmeldenummer: PCT/AT2016/000034
(87) Internationale Veröffentlichungsnummer: WO 2017/054018

(56) Entgegenhaltungen:
- WO-A1-2015/131891
- WO-A2-01/89576
- ANN HUYGENS ET AL: "In vivo accumulation of different hypericin ion pairs in the urothelium of the rat bladder", BJU INTERNATIONAL, Bd. 95, Nr. 3, 1. Februar 2005 (2005-02-01), Seiten 436-441, XP055285893, GB ISSN: 1464-4096, DOI: 10.1111/j.1464-410X.2005.05316.x
- ANDREAS KUBIN ET AL: "Fluorescence Diagnosis of Bladder Cancer with New Water Soluble Hypericin Bound to Polyvinylpyrrolidone: PVP-Hypericin", PHOTOCHEMISTRY AND PHOTOBIOLOGY, Bd. 84, Nr. 6, 1. November 2008 (2008-11-01), Seiten 1560-1563, XP055285844, US ISSN: 0031-8655, DOI: 10.1111/j.1751-1097.2008.00384.x in der Anmeldung erwähnt
- MICHAEL STRAUB ET AL: "A Phase IIA Dose-Finding Study of PVP-Hypericin Fluorescence Cystoscopy for Detection of Nonmuscle-Invasive Bladder Cancer", JOURNAL OF ENDOUROLOGY, Bd. 29, Nr. 2, 1. Februar 2015 (2015-02-01), Seiten 216-222, XP055285861, US ISSN: 0892-7790, DOI: 10.1089/end.2014.0282

## Beschreibung

Die Erfindung betrifft eine Formulierung zum Herstellen einer Instillationslösung zur Verwendung bei der photodynamischen Diagnose, enthaltend an einen polymeren Komplexbildner, nämlich ein Polyethylenglykol oder ein poly-N-Vinylamid gebundenes Hypericin in Form eines Alkalimetallsalzes, insbesondere des Kaliumsalzes oder des Natriumsalzes, gemäß den Ansprüchen.

Blasenkrebs ist die häufigste Krebserkrankung der Harnwege. Blasenkrebs betrifft Männer mehr als dreimal so häufig als Frauen. Es ist die siebent häufigste diagnostizierte Krebsart bei Männern (Ferlay et al., 2013). Etwa 75-85% der Patienten mit neu diagnostiziertem Blasenkrebs zeigen nicht-muskelinvasive Blasentumore, d.h. Tumore, die auf die Schleimhaut beschränkt sind. Es handelt sich dabei um die Tumorstadien Carcinoma in situ (Tis), Ta, oder T1 (Babjuk et al., 2015). Die Rezidivraten bei nicht-muskelinvasivem Blasenkarzinom sind sehr häufig. Die Wahrscheinlichkeit des Wiederauftretens liegt bei 15 bis 61% innerhalb des ersten Jahres und 31 bis 78% nach 5 Jahren (Witjes, Douglass, 2007). Die hohen Rezidivraten erfordern eine jahrelange Überwachung und Nachbeobachtung einmal erkrankter Patienten.

Das häufigste Symptom von nicht-muskelinvasivem Blasenkrebs ist Hämaturie. Daneben können auch irritative Symptome oder Schmerzen in den unteren Harnwegen auftreten. Eine körperliche Untersuchung gibt keinen Aufschluss über einen potentiellen nicht-muskelinvasiven Blasentumor (Babjuk et al., 2015). Die visuelle Inspektion der Blase mit einem Endoskop und Weißlichtbeleuchtung (Weißlicht-Zystoskopie) und eine Entnahme von Gewebeproben stellt eine erste Diagnose dar. Diese Methode ist zuverlässig für exophytische Tumore. Flache Karzinome (insbesondere Tis), Dysplasien, multifokales Wachstum und mikroskopische Läsionen sind sehr viel schwieriger zu erkennen und werden bei einer Weißlicht-Zystoskopie oft übersehen.

Die Methode der Fluoreszenz-Zystoskopie (auch als Photodynamische Diagnostik (PDD) bezeichnet) verbessert die Erkennungsraten von nicht-muskelinvasivem Blasenkrebs, insbesondere von Tis, und vermindert somit die Rezidivrate (Burger et al., 2013; Kausch et al., 2010; Stenzel et al., 2010) .

Die photodynamische Diagnostik (PDD) nutzt die photoaktiven Eigenschaften bestimmter Verbindungen, sogenannter Photosensibilisatoren, die sich bevorzugt im Tumorgewebe anreichern und die optische Abgrenzung zwischen normalem und neoplastischem Gewebe verbessern.

Das Grundprinzip der photodynamischen Diagnostik (PDD) basiert auf einem Zwei-Schritt-Verfahren, umfassend eine systemische oder topische Anwendung eines Photosensibilisators und die Aktivierung des Photosensibilisators durch Bestrahlen mit sichtbarem Licht mit einer geeigneten Wellenlänge.

Der "Goldstandard" bei der Detektion von nicht-muskelinavsivem Blasenkrebs ist die Weißlicht-Zystoskopie. Bei Verdacht auf Tis wird jedoch die Verwendung einer Fluoreszenz-Zystoskopie empfohlen (Babjuk et al., 2015), wodurch durchschnittlich 20% mehr Tis gefunden werden können (Witjes et al., 2010).

Die Porphyrin Precurser 5-Aminolevulinsäure (5-ALA) und das Derivat Hexaminolevulinsäure (HAL) werden in der Fluoreszenzdiagnostik eingesetzt. Beide Substanzen sind Prodrugs. Durch Metabolisierung der Pro-drug entsteht ein photoaktives Molekül, das zur PDD genutzt wird. Die einzige als Arzneimittel zugelassene Substanz für die PDD in der Indikation Blasenkarzinom ist die Hexaminolevulinsäure (Hexvix®, Cysview®).

Die Eigenschaften von Hypericin (1,3,4,6,8,13-Hexahydroxy-10,11-dimenthylphenanthro (1,10,9,8-opqra) perylene-7,14-dion) als Photosensibilisator und Indikator für Krebszellen, speziell zum Nachweis von nicht-muskelinvasiven Tumoren des Urothels, sind bekannt. Hypericin ist keine Pro-drug und muss im Gewebe nicht metabolisiert werden, sondern kann direkt mit Licht mit einer geeigneten Wellenlänge angeregt werden, sobald das Hypericin im Gewebe akkumuliert ist.

Allerdings ist reines Hypericin hydrophob und in Wasser unlöslich. Aus diesem Grund wurden in der Vergangenheit in präklinischen Studien ein wasserlösliches Polymer, Polyethylenglycol (PEG), oder in klinischen Studien Serumproteine als wirksame Hypericin-Transporter / Träger verwendet, um das unlösliche Hypericin in die Zielzellen zu bringen (D'Hallewin et al., 2000 und 2002; Olivo et al., 2003; Pytel et al., 2002).

Die Löslichkeit von Hypericin kann durch die Gegenwart des Hilfsstoffes Polyvinylpyrrolidon ("Povidon", PVP) erhöht werden (WO 01/89576 A2).

Eine Formulierung aus 25 mg PVP und 0.25 mg Hypericin wurde klinisch an 57 Patienten untersucht (Kubin et al., 2008). Bezüglich flacher Läsionen (Tis und Dysplasien) wurde auf Läsionsebene eine Detektionsrate von 100% hinsichtlich Tis und 85% hinsichtlich Dysplasien mit PVP-Hypericin unterstützter PDD erreicht, wogegen unter Weißlicht-Zystoskopie nur 33% (Tis) und 31% (Dysplasien) detektiert wurden.

Die verbesserte Detektion auf Läsionsebene zeigt sich auch auf der Patientenebene: bei 16% der Patienten detektiert die PVP-Hypericin unterstützte PDD Läsionen, die in der Weißlicht-Zystoskopie übersehen wurden. Die Instillationszeit (Verweildauer in der Blase) der PVP-Hypericinlösung lag bei 60-220 Minuten (im Durchschnitt 111 ± 39 (SD) Minuten (Kubin et al., 2008).

Auch wenn die bekannte PDD-Studie gute Ergebnisse erzielt hat, bleibt ein gravierendes Problem ungelöst. Die lange Instillationszeit (das bedeutet das Halten der verabreichten Lösung in der Blase des Patienten) von mindestens 60 Minuten stellt eine Belastung für Patienten mit nicht-muskelinvasivem Blasenkarzinom, die sehr oft unter Schmerzen oder Krämpfen leiden, dar.

Die WO 2015/131891 A1 betrifft betrifft die Verwendung von Hypericin in Form des Natriumsalzes sowohl für die photodynamische Diagnose als auch für die photodynamische Therapie.

Das in WO 2015/131891 A1 geoffenbarte Produkt liegt in Form einer wasserfreien Lösung in Alkohol (nämlich Ethanol) vor und enthält als Lösungsvermittler PEG. Die Lösung ist vor ihrer Verwendung als Photosensitizer in einer Trägerlösung (Glukoselösung) zu verdünnen.

Die so erhaltene Infusionslösung soll 0,01 bis 0,05 mg Natrium-Hypericinat enthalten und soll über einen Zeitraum von 90 Minuten administriert werden.

Der Erfindung liegt die Aufgabe zugrunde, eine sterile pharmazeutische Formulierung von Hypericin zur Verfügung zu stellen, die großtechnisch herstellbar ist und eine entsprechende Langzeitstabilität aufweist. Diese Formulierung von Hypericin soll als Diagnostikum für Blasenkrebs problemloser als bekannte Formulierungen verwendbar sein.

Gelöst wird die oben genannte Aufgabe mit einer Formulierung von Hypericin, welche die Merkmale von Anspruch 1 aufweist.

Bevorzugte und vorteilhafte Ausführungsformen der erfindungsgemäßen Formulierung sind Gegenstand der Unteransprüche.
Überraschenderweise hat sich gezeigt, dass die erfindungsgemäße Formulierung von Hypericin nur dann stabil und damit unter klinischen Bedingungen anwendbar ist, wenn Hypericin als Salz vorliegt.

Im Rahmen einer klinischen Studie hat sich gezeigt, dass die erfindungsgemäße Formulierung von Hypericin für die Detektion von malignen Läsionen in Patienten mit Verdacht auf nichtmuskelinvasives Blasenkarzinom besonders gut geeignet ist. Überdies erlaubt die erfindungsgemäße Formulierung von Hypericin ohne Beeinträchtigung der Ergebnisse der PDD erheblich kürzere Instillationszeiten, was eine vorteilhafte Verringerung der Belastung der Patienten bedeutet.

Überraschenderweise hat sich eine Anwendung der erfindungsgemäßen Formulierung mit einer Dosis von 22.5 mg PVP und 0.225 mg Hypericin in Kombination mit einer Instillationszeit von 30 bis maximal 50 Minuten für eine PDD des nicht-muskelinvasiven Blasenkarzinoms als optimale Dosis erwiesen.

Mit Hilfe der erfindungsgemäßen Formulierung konnten bei einer Instillationszeit von 30 bis 50 Minuten in 35% der Patienten Tis-Läsionen identifiziert werden, die mit der Weißlicht-Zystoskopie übersehen worden sind.

Eine vollständige Entfernung des Tumors (Resektion) stellt den ersten wichtigen Schritt für die Therapie der Patienten dar. Oft ist eine komplette Resektion des Tumorgewebes schwierig. Das hat zur Folge, dass Tumormaterial übersehen wird und in der Blase des Patienten verbleibt. Aus diesem Grund ist es besonders wichtig, die Ränder und Grenzen des Tumorgewebes vollständig zu detektieren und zu entfernen.

Die Anwendung der erfindungsgemäßen Formulierung mit einem Hypericin-Gehalt von 0.225 mg (Beispiel 1) zeigte eine bessere Erkennbarkeit der Gewebsdetails, im Besonderen hinsichtlich der Randbereiche der Tumoren. Dadurch wird eine Diskriminierung zwischen malignem und benignem Gewebe erleichtert und der Tumor kann vollständig entfernt werden.

Diese bessere Differenzierung zwischen malignem und benignem Gewebe ist ebenfalls mit einem Hypericin-Gehalt von 0.500 mg und einer Instillationszeit von nur 15 Minuten zu erreichen.

Nachdem Tis-Läsionen mit extrem hohen Rezidivraten und einer sehr hohen Wahrscheinlichkeit der Progression (das bedeutet die Entwicklung eines Tumors in ein weiter fortgeschrittenes Stadium oder das Auftreten von Metastasen) assoziiert sind, stellen eine verbesserte Detektionsrate und damit eine vollständige Entfernung des Tumors mit Hilfe der erfindungsgemäßen Formulierung von Hypericin einen signifikanten Vorteil bezüglich des weiteren Krankheitsverlaufes dar.

Die bei der Verwendung der erfindungsgemäßen Formulierung erforderliche Instillationszeit von 15, durchschnittlich 30 bis maximal 50 Minuten ist bedeutend kürzer als jene Instillationszeit, die für eine PDD mit Hexaminolevulinsäure verwendet wird. Bereits nach einer Instillationszeit von durchschnittlich 30 Minuten konnten mit Hilfe der genannten Hypericin-Formulierung maligne Läsionen diagnostiziert werden. Diese deutlich verkürzte Instillationszeit stellt eine große Erleichterung für Patienten mit nicht-muskelinvasivem Blasenkarzinom dar und erhöht die Wahrscheinlichkeit der Einhaltung der erforderlichen Einwirkungszeit.

Nachstehend werden Beispiele für die erfindungsgemäße Formulierung von Hypericin beschrieben:
Allgemeine Verfahrensweise zum Herstellen einer Formulierung mit dem Wirkstoff Natrium Hypericinat:
Ziel ist das Herstellen einer Hypericin enthaltenden Formulierung zur Anwendung als Photosensibilisator im Bereich der photodynamischen Diagnostik.

Die erfindungsgemäße Formulierung wird aus einem Salz von Hypericin, insbesondere aus Na-Hypericinat, hergestellt.

Um den Hypericin-Gehalt des Ausgangsmaterials zu definieren, werden neben der Gehaltsbestimmung vor allem Wassergehalt und im Falle von Natrium-Hypericinat der Natrium Anteil festgehalten. Die chemisch-physikalischen Eigenschaften können einen Einfluss auf die Formulierung des pharmazeutischen Arzneimittels haben.

Für klinische Anwendung ist eine Stabilität der erfindungsgemäßen Formulierung erforderlich. Die Stabilität wird durch die Zusammensetzung des Fertigproduktes gewährleistet und betrifft gleichzeitig auch das Herstellungsverfahren. Durch die verwendeten Puffersysteme kann auch während des Herstellens bis zum Lyophilisieren des Fertigprodukts eine hinreichende Stabilität der Bulklösung erreicht werden.

Als Puffersysteme können verschiedene Zusatzstoffe herangezogen werden, die bevorzugt sowohl für die Bulklösung als auch für die rekonstituierte Lösung einen physiologisch verträglichen pH-Wert und einen osmotischen Druck nach der Rekonstitution mit 50 ml Aqua ad Injectabilia von 290 mOsmol/kg erzielen. Phosphat- oder Zitrat-Puffersysteme können in erster Linie zum Einsatz kommen.

Nach dem Fertigstellen der Bulklösung aus den oben genannten Bestandteilen wird die entsprechende Menge der Bulklösung in Injektionsflaschen abgefüllt und lyophilisiert.

### Beispiel 1:

Aus dem Na-Hypericinat wird eine Lösung mit einer Zieleinwaage von 27,0 mg Hypericin hergestellt.

Zu 562,5 mg PVP k25 werden 5,0 g der Hypericin-Lösung zugesetzt und vollständig gelöst.

Diese Lösung wird quantitativ mit einer Phosphatpufferlösung auf 250,0 g aufgefüllt. Die Endkonzentration dieser Lösung ist 0,0225 mg Hypericin/g Lösung.

Für das Lyophilisieren wird eine definierte Menge der so erhaltenen Bulklösung in Injektionsflaschen abgefüllt und mit einem entsprechenden Lyo-Programm das fertige Lyophilisat hergestellt.

### Beispiel 2:

Es wird, so wie in Beispiel 1 angegeben, gearbeitet, wobei statt PVP k25 zum Komplexieren vom Na-Hypericinat PVP k17 verwendet wird.

### Beispiel 3:

Es wird, so wie in Beispiel 1 angegeben, gearbeitet, wobei statt PVP k25 zum Komplexieren vom Na-Hypericinat PVP k30 verwendet wird.

### Beispiel 4:

Es wird, so wie im Beispiel 1, 2 oder 3 angegeben, gearbeitet, wobei statt der Phosphatpufferlösung eine Zitronensäure-Pufferlösung verwendet wird.

Die wie in den Beispielen 1 bis 4 beschrieben hergestellten Bulklösungen können mit unterschiedlichen Hypericin-Gehalten produziert werden.

Aus der Hypericin-Stammlösung (hergestellt aus Natrium-Hypericinat) können vor dem weiteren Verarbeiten folgende Verdünnungen hergestellt werden:
- Zu 0,4 g der in Beispiel 1 genannten Hypericin Lösung wird eine definierte Menge an Lösungsmittel dazugegeben und homogenisiert. In diesem Fall werden im weiteren Schritt nur 187,5 mg PVP (unterschiedliche Typen von PVP sind möglich) zur Komplexbildung dazugegeben. Damit erreicht man in der fertigen Bulklösung eine Endkonzentration von 0,0075 mg Hypericin/g Lösung.
- Zu 0,2 g der in Beispiel 1 genannten Hypericin-Lösung wird eine definierte Menge an Lösungsmittel dazugegeben und homogenisiert. In diesem Fall werden im weiteren Schritt nur 62,5 mg PVP (unterschiedliche Typen von PVP sind möglich) zur Komplexbildung dazu gegeben. Damit erreicht man in der fertigen Bulklösung eine Endkonzentration von 0,0025 mg Hypericin/g Lösung.

In einer klinischen Studie wurde die Formulierung von Hypericin gemäß Beispiel 1 mit einer Dosis von 22,5 mg PVP und 0,225 mg Hypericin für eine Zeitdauer von durchschnittlich 30 bis 35 Minuten, maximal bis zu 50 Minuten, in die Blase von Patienten instilliert. Danach wurde eine Zystoskopie, zuerst unter Weißlicht, danach unter Fluoreszenzlicht, vorgenommen. Verdächtige Läsionen wurden entnommen und mittels histologischer Untersuchung klassifiziert.

In insgesamt 20 Patienten konnten Tis-Läsionen detektiert und histologisch bestätigt werden. Die eine erfindungsgemäße Formulierung von Hypericin verwendende PDD zeigt dabei einen entscheidenden Vorteil in der Detektion von Tis in 35% der Patienten. In diesen Patienten wären die Tis-Läsionen ohne PDD, welche die erfindungsgemäße Formulierung von Hypericin benützt, unentdeckt geblieben.

### Referenzen:

Babjuk M, Böhle A, Burger M, Comperat E, Kaasinen E, Palou J, Rouprêt M, van Rhijn BWG, Shariat S, Sylvester S, and Zigeuner R. European Association of Urology Guidelines 2015 edition: Guidelines on Non-muscle-invasive Bladder Cancer (Ta, T1 and CIS)
Burger M, Grossman HB, Droller M, Schmidbauer J, Hermann G, Dr Goescu O, Ray E, Fradet Y, Karl A, Burgues JP, Witjes JA, Stenzl A, Jichlinski P, Jocham D. Photodynamic diagnosis of non-muscle-invasive bladder cancer with hexaminolevulinate cystoscopy: a meta-analysis of detection and recurrence based on raw data. Eur Urol. 2013 Nov; 64(5):846-854
D'Hallewin MA, de Witte P A, Waelkens E, Merlevede W, Baert L Fluorescence detection of flat bladder carcinoma in situ after intravesical instillation of Hypericin. J. Urol. 2000; 164 (2) :349-351
D'Hallewin MA, Kamuhabwa AR, Roskams T, de Witte PAM, Baert L. Hypericin-based fluorescence diagnosis of bladder carcinoma. BJU International 2002; 89:760-763
D'Hallewin MA, Bezdetnaya L, Guillemin F. Fluorescence detection of bladder cancer: A review. Eur Urol. 2002; 42(5):417-425
Ferlay J, Steliarova-Foucher E, Lortet-Tieulent J, Rosso S, Coebergh JW, Comber H, Forman D, Bray F. Cancer incidence and mortality patterns in Europe: estimates for 40 countries in 2012. Eur J Cancer. 2013 Apr;49(6):1374-1403
Kausch I, Sommerauer M, Montorsi F, et al.: Photodynamic diagnosis in non-muscleinvasive bladder cancer: a systematic review and cumulative analysis of prospective studies. Eur Urol. Apr 2010; 57 (4) :595-606
Kubin A, Meissner P, Wierrani F, Burner U, Bodenteich A, Pytel A, Schmeller N. Fluorescence diagnosis of bladder cancer with new water soluble hypericin bound to polyvinylpyrrolidone: PVP-Hypericin. Photochem Photobiol. 2008; 84(6):1560-1563
Olivo M, Lau W, Manivasager V, Tan PH, Soo K C, Cheng C. Macromicroscopic fluorescence of human bladder cancer using Hypericin fluorescence cystoscopy and laser confocal microscopy. Int J Oncol 2003; 23(4):983-990
Pytel A, Schmeller N. New aspect of photodynamic diagnosis of bladder tumors: fluorescence cytology. Urology 2002; 59:216-219
Stenzl A, Burger M, Fradet Y, Mynderse LA, Soloway MS, Witjes JA, Kriegmair M, Karl A, Shen Y, Grossman HB. Hexaminolevulinate guided fluorescence cystoscopy reduces recurrence in patients with nonmuscle invasive bladder cancer. J Urol. Nov 2010; 184 (5) :1907-1913
Witjes JA, Douglass J. The role of hexaminolevulinate fluorescence cystoscopy in bladder cancer. Nat Clin Pract Urol. 2007 Oct;4(10) :542-549
Witjes JA, Redorta JP, Jacqmin D, Sofras F, Malmström PU, Riedl C, Jocham D, Conti G, Montorsi F, Arentsen HC, Zaak D, Mostafid AH, Babjuk M. Hexaminolevulinate-guided fluorescence cystoscopy in the diagnosis and follow-up of patients with non-muscle-invasive bladder cancer: review of the evidence and recommendations. Eur Urol. 2010 Apr;57(4):607-614

## Patentansprüche

1. Formulierung zum Herstellen einer Instillationslösung, die bei der photodynamischen Diagnose verwendbar ist, enthaltend an einen polymeren Komplexbildner, nämlich ein Polyethylenglykol oder ein poly-N-Vinylamid gebundenes Hypericin in Form eines Alkalimetallsalzes, insbesondere des Kaliumsalzes oder des Natriumsalzes, wobei die Instillationslösung 0,225 mg Hypericin enthält, **dadurch gekennzeichnet, dass** die Formulierung ein stabiles Lyophilisat ist, das aus einer wässerigen Lösung, enthaltend das Alkalimetallsalz von Hypericin, den Komplexbildner und ein Puffersystem, insbesondere einen Phosphatpuffer oder eine Zitronensäure-Pufferlösung, gewonnen ist.

2. Formulierung nach Anspruch 1, **dadurch gekennzeichnet, dass** das Poly-N-Vinylamid Polyvinylpyrrolidon (PVP)verschiedenen Polymerisations- und Vernetzungsgrades ist.

3. Verfahren zum Herstellen einer stabilen Formulierung nach Anspruch 1 oder 2, **gekennzeichnet durch** nachstehende, aufeinanderfolgende Verfahrensschritte,
a) es wird eine wässerige Lösung des Alkalimetallsalzes von Hypericin hergestellt,
b) die in Schritt a) erhaltene Lösung wird zu dem Komplexbildner, insbesondere Polyvinylpyrrolidon zugegeben und gelöst,
c) zu der in Schritt b) erhaltenen Lösung wird ein Phosphatpuffer oder eine Zitronensäure-Pufferlösung zugesetzt, um eine Konzentration von 0,0225 mg Hypericin/g Lösung zu erhalten und
d) Anteile der nach Schritt c) erhaltenen Bulk-Lösung werden lyophilisiert.

## Claims

1. Formulation for preparing an instillation solution useful in photodynamic diagnosis, containing hypericin in the form of an alkali metal salt, in particular the potassium salt or the sodium salt, bonded to a polymeric complexing agent, namely a polyethylene glycol or a poly-N-vinylamide, wherein the instillation solution contains 0.225 mg hypericin, **characterized in that** the formulation is a stable lyophilisate obtained from an aqueous solution containing the alkali metal salt of hypericin, the complexing agent and a buffer system, in particular a phosphate buffer or a citric acid buffer solution.

2. Formulation according to claim 1, **characterized in that** the poly-N-vinylamide is polyvinylpyrrolidone (PVP) of different degrees of polymerization and crosslinking.

3. Method for preparing a stable formulation according to claim 1 or 2, **characterized by** the following successive method steps,
(a) an aqueous solution of the alkali metal salt of hypericin is prepared,
(b) the solution obtained in step (a) is added to the complexing agent, in particular polyvinylpyrrolidone, and dissolved,
(c) a phosphate buffer or a citric acid buffer solution is added to the solution obtained in step b) to obtain a concentration of 0.0225 mg hypericin/g solution and
(d) fractions of the bulk solution obtained in step (c) are lyophilized.

## Revendications

1. Formule pour la fabrication d'une solution pour instillation, qui peut être utilisée dans le diagnostic photodynamique, comprenant de l'hypéricine liée à un agent complexant polymère, à savoir un polyéthylène glycol ou un poly-N-vinylamide sous la forme d'un sel de métal alcalin, notamment de sel de potassium ou de sel de sodium, la solution pour instillation comprenant 0,225 mg d'hypéricine, **caractérisée en ce que** la formule est un lyophilisat stable, qui est obtenu à partir d'une solution aqueuse, comprenant le sel de métal alcalin d'hypéricine, l'agent complexant et un système tampon, notamment un tampon phosphate ou une solution tampon à l'acide citrique.

2. Formule selon la revendication 1, **caractérisée en ce que** le poly-N-vinylamide est une polyvinylpyrrolidone (PVP) de différents degrés de polymérisation et de réticulation.

3. Procédé pour établir une formule stable selon la revendication 1 ou 2, **caractérisé par** les étapes de procédure successives suivantes,
a) une solution aqueuse est produite à partir du sel de métal alcalin d'hypéricine,
b) la solution obtenue à l'étape a) est ajoutée à et dissoute dans l'agent complexant, notamment la polyvinylpyrrolidone,
c) un tampon phosphate ou une solution tampon à l'acide citrique est ajouté à la solution obtenue à l'étape b) afin d'obtenir une concentration de 0,0225 mg d'hypéricine/g de solution et
d) des portions de la solution en vrac obtenue à l'étape c) sont lyophilisées.
